# EUROPEAN PATENT APPLICATION

(11) **EP 1 555 271 A1**
(43) Date of publication of application: **20.07.2005**
(21) Application number: 04000772.6
(22) Date of filing: 15.01.2004
(51) Int. Cl.: C07K 14/705, C07K 19/00, C07K 16/28, C12N 15/12, C12N 15/63, G01N 33/53, G01N 33/68, A61K 38/17, A61K 39/00, A61K 39/395

(54) **Peptides useful for diagnosis and therapy of TRP-2+ and/or TRP-1+ tumors**

(71) Applicant: Deutsches Krebsforschungszentrum Stiftung des öffentlichen Rechts, 69120 Heidelberg (DE)
(72) Inventor: Schadendorf, Dirk, 68165 Mannheim (DE); Paschen, Annette, 68549 Ilvesheim (DE); Kropshofer, Harald, 79539 Lörrach (DE)
(74) Representative: Schüssler, Andrea, Dr.

(57) **Abstract**

Described are the peptide motifs QCTEVRADTRPWSGP (human TRP-2₆₀₋₇₄), QCTEYRADTTPWSGP (TRP-2₆₀₋₇₄YT, epitope variant of human TRP-2₆₀₋₇₄), LPYWNFATGRNECDV (human TRP-2₂₄₂₋₂₅₆), SEEILGPDGNTPQFE (human TRP-1₁₆₆₋₁₈₀), LRRYNADISTFPL (human TRP-1₄₁₅₋₄₂₇) and related motifs characterizing peptides which are capable of activating CD4+ T helper cells (Th) against a TRP-2⁺ and/or TRP-1⁺ tumor, preferably a malignant melanoma or glioma. Moreover, various diagnostic and therapeutic uses of peptides containing said motifs and antibodies directed against said peptides are described.

SEQUENCE LISTING

<110>  Deutsches Krebsforschungszentrum Stiftung des öffentlichen
       Rechts

<120>  Peptides useful for diagnosis and therapy of TRP-2 and/or TRP-1
       tumors

<130>  K 3168EP

<140>  EP 04000772.6
<141>  2004-01-15

<160>  17

<170>  PatentIn version 3.2

<210>  1
<211>  15
<212>  PRT
<213>  Homo sapiens

<400>  1

Gln Cys Thr Glu Val Arg Ala Asp Thr Arg Pro Trp Ser Gly Pro
1               5                   10                  15


<210>  2
<211>  15
<212>  PRT
<213>  Homo sapiens

<400>  2

Gln Cys Thr Glu Tyr Arg Ala Asp Thr Thr Pro Trp Ser Gly Pro
1               5                   10                  15


<210>  3
<211>  15
<212>  PRT
<213>  Homo sapiens

<400>  3

Leu Pro Tyr Trp Asn Phe Ala Thr Gly Arg Asn Glu Cys Asp Val
1               5                   10                  15


<210>  4
<211>  15
<212>  PRT
<213>  Homo sapiens

<400>  4

Ser Glu Glu Ile Leu Gly Pro Asp Gly Asn Thr Pro Gln Phe Glu
1               5                   10                  15


<210>  5
<211>  13
<212>  PRT
<213>  Homo sapiens

<400>  5

Leu Arg Arg Tyr Asn Ala Asp Ile Ser Thr Phe Pro Leu
1               5                   10

<210>  6
<211>  15
<212>  PRT
<213>  Homo sapiens

<400>  6

Gln Cys Thr Glu Tyr Arg Ala Asp Thr Thr Pro Trp Ser Gly Pro
1               5                   10                  15

<210>  7
<211>  21
<212>  DNA
<213>  Artificial sequence

<220>
<223>  Primer

<400>  7
cagttccccc gagtctgcat g                                              21

<210>  8
<211>  24
<212>  DNA
<213>  Artificial sequence

<220>
<223>  Primer

<400>  8
agtttcttca actgaaactg gcag                                           24

<210>  9
<211>  15
<212>  PRT
<213>  Artificial sequence

<220>
<223>  Tetanus toxin

<400>  9

Met Gln Tyr Ile Lys Ala Asn Ser Lys Phe Ile Gly Ile Thr Glu
1               5                   10                  15

<210>  10
<211>  13
<212>  PRT
<213>  Influenza virus

<400>  10

Pro Lys Tyr Val Lys Gln Asn Thr Leu Lys Leu Ala Thr
1               5                   10

<210>  11
<211>  15
<212>  PRT
<213>  Homo sapiens

<400> 11

His Leu Leu Cys Leu Glu Arg Asp Leu Gln Arg Leu Ile Gly Asn
1               5                   10                  15


<210> 12
<211> 15
<212> PRT
<213> Homo sapiens

<400> 12

Ser Leu Asp Asp Tyr Asn His Leu Val Thr Leu Cys Asn Gly Thr
1               5                   10                  15


<210> 13
<211> 16
<212> PRT
<213> Homo sapiens

<400> 13

Phe Thr Asp Ala Ile Phe Asp Glu Trp Met Lys Arg Phe Asn Pro Pro
1               5                   10                  15


<210> 14
<211> 15
<212> PRT
<213> Homo sapiens

<400> 14

Asn Leu Leu Asp Leu Ser Lys Glu Glu Lys Asn His Phe Val Arg
1               5                   10                  15


<210> 15
<211> 15
<212> PRT
<213> Homo sapiens

<400> 15

His Leu Leu Arg Leu Glu Lys Asp Met Gln Glu Met Leu Gln Glu
1               5                   10                  15


<210> 16
<211> 13
<212> PRT
<213> Homo sapiens

<400> 16

Leu Arg Arg Tyr Asn Ala Asp Ile Ser Thr Phe Pro Leu
1               5                   10


<210> 17
<211> 15
<212> PRT
<213> Mus musculus

<400> 17

Gln Cys Ala Glu Val Gln Thr Asp Thr Arg Pro Trp Ser Gly Pro
1               5                   10                  15

## Description

The present invention relates to peptide motifs QCTEVRADTRPWSGP (human TRP-2₆₀₋₇₄), QCTEYRADTTPWSGP (TRP-2₆₀₋₇₄YT, epitope variant of human TRP-2₆₀₋₇₄), LPYWNFATGRNECDV (human TRP-2₂₄₂₋₂₅₆), SEEILGPDGNTPQFE (human TRP-1₁₆₆₋₁₈₀), LRRYNADISTFPL (human TRP-1₄₁₅₋₄₂₇) and related motifs characterizing TRP-2 and TRP-1 derived peptides which are capable of activating CD4⁺ T helper cells (Th) against a TRP-2⁺ and/or TRP-1⁺ tumor, preferably a malignant melanoma or glioma. The present invention also relates to various diagnostic and therapeutic uses of peptides and (recombinant) proteins containing said motifs and (recombinant) RNA, (recombinant) DNA and recombinant viruses encoding peptides or proteins containing said motifs. The present invention also relates to the therapeutic use of T cells specifically recognizing that peptides in complex with HLA-class II molecules and antibodies directed against said peptides.

In vivo sensitized T cells isolated from the peripheral blood and tumor tissue of melanoma patients have repeatedly been demonstrated to recognize epitopes derived from melanoma differentiation antigens (MDA). These MDA-specific T cells exhibit cytolytic activity against autologous tumors in vitro and, most importantly, also in vivo, as demonstrated by two recent clinical studies of adoptive T cell transfer, in which patients were treated with in vitro expanded autologous MDA-specific CD8⁺ T cell clones and bulk T cell cultures, respectively.

Two members of the MDA family, the differentiation antigens TRP-2 (Tyrosinase-related protein-2) and TRP-1 (Tyrosinase-related protein-1) have been selected as target antigens to demonstrate the protective and therapeutic capacity of diverse T cell-based vaccination strategies in the B16 autologous melanoma model. In addition, TRP-2- and TRP-1-reactive T cells were identified as mediators of anti-melanoma effects induced by complex tumor-cell based vaccines. Quantification of TRP-2 expression by reverse transcriptase RealTime PCR in human melanomas revealed the presence of TRP-2 mRNA in nearly 90% of metastatic melanoma tumors. Interestingly, elevated copy levels of TRP-2 mRNA have been demonstrated to significantly correlate with improved overall survival of stage IV melanoma patients. One might speculate that the anti-tumor activity of cytotoxic TRP-2-specific T cells contributes to this improvement of survival. Expression of TRP-2 and TRP-1 can also be detected in human gliomas indicating that the clinical application of TRP-2- and TRP-1-based vaccines might be broadened from melanoma to glioma.

Cytotoxic CD8⁺ T cells isolated either from the blood or tumor tissue of melanoma patients have been employed for the identification of HLA-class I restricted TRP-2 and TRP-1 epitopes presented by HLA-A2 (Parkhurst et al., Cancer Res. 58 (1998), 4895-901; Noppen et al., Int. J. Cancer 87 (2000), 241; Sun et al. , In. J. Cancer 87 (2000), 399; Harada et al., Cancer Res. 61 (2001), 399; Sun et al., Cancer Res. 62 (2002), 2875), HLA-A31, -A33 (Wang et al., J. Exp. Med. 184 (1996), 2207; Wang et al., J. Immunol. 160 (1998) and HLA-Cw8 (Castelli et al., J. Immunol. 162 (1999), 1739) molecules, respectively, providing the basis for the development and evaluation of antigen-specific T cell-based immunotherapies. Although CD8⁺ cytotoxic T cells (CTL) are still classified as the main mediators of antigen-specific tumor destruction, it is now well accepted that effective anti-tumor immunity requires the participation of both, antigen-specific CD8⁺ and CD4⁺ T helper cells. CD4⁺ T cells are involved in several steps of the induction and maintenance of CTL immunity: The interaction of CD40L on activated antigen-specific CD4⁺ T cells with CD40 on DC greatly increases the DC's capacity to effectively prime CD8⁺ T cells (Schoenberger et al., Nature 393 (1998), 480; Bennett et al., Nature 393 (1998), 478). In addition, signals delivered by antigen-specific CD4⁺ T cells are essentially needed for the generation of CD8⁺ T cells memory (Janssen et al., Nature 421 (2003), 852-6). Therefore, antigen-specific vaccines should stimulate both T cell subsets.

So far, specific immunotherapeutic treatment of melanoma patients was mainly restricted to the use of peptide vaccines or peptide DC-vaccines which were merely based on CTL epitope sequences. This was due to the fact that while HLA-class I presented TRP-2 epitopes and TRP-1 epitopes are characterized and can be introduced into antigen-specific vaccination strategies, information about HLA-class II restricted epitopes is lacking. Thus, so far tumor patients are treated with vaccines merely capable of stimulating specific CTL. These vaccines cannot be used for targeted activation of specific Th.

Therefore, it is the object of the present invention to provide a means for an improved immunotherapeutic treatment of tumor patients which overcomes the disadvantages of the therapeutic approaches presently used and to provide a means for improved monitoring of specific immunological responses in tumor patients.

According to the present invention this is achieved by the subject matters defined in the claims.

During the experiments leading to the present invention computer algorithms and HLA-class II transgenic mice were used as tools for the identification of TRP-2- and TRP-1-derived Th epitopes. From three predicted potential HLA-DRB1*0301 TRP-2 epitopes two exhibited HLA-DRB1*0301 binding capacity. One of the peptides, TRP-2₆₀₋₇₄, was clearly immunogenic in HLA-DR3tg mice and protein immunization revealed processing of this epitope from global antigen. Importantly, in vivo sensitized T cells specific for TRP-2₆₀₋₇₄ could also be detected in the peripheral blood from a HLA-DRB1*0301⁺ melanoma patient verifying TRP-2 ₆₀₋₇₄ as a target epitope of spontaneous tumor-induced T cell responses. TRP-2₆₀₋₇₄-specific T cell responses could also be induced by primary in vitro stimulation of peripheral blood mononuclear cells (PBMC) from normal HLA-DRB1*0301⁺ donors. Further binding studies revealed that the TRP-2₆₀₋₇₄ epitope also binds to the HLA-DRB1*0401 molecule. In vivo sensitized T cells specific for TRP-2₆₀₋₇₄ and could also be detected in the peripheral blood from a HLA-DRB1*0401⁺ melanoma patient again verifying TRP-2₆₀₋₇₄ as a target epitope of spontaneous T cell reactivity. Interestingly, T cells from this patient also recognized a variant of the TRP-2₆₀₋₇₄ epitope, designated TRP-2₆₀₋₇₄YT, with even higher reactivity. Primary T cell responses against this epitope variant TRP-2₆₀₋₇₄YT could be induced by in vitro stimulation of PBMC from normal HLA-DRB1*0401⁺ donors and these T cells TRP-2₆₀₋₇₄YT cross reacted with the natural TRP-2₆₀₋₇₄ epitope. From three predicted TRP-1 derived sequences two exhibited HLA-DRB1*0301 binding capacity. Both peptides, TRP-1₁₆₆₋₁₈₀ and TRP-1₂₂₇₋₂₄₁, were clearly immunogenic in HLA-DR3 transgenic mice. Most importantly, in vivo sensitized T cells specific for TRP-1₁₆₆₋₁₈₀ could also be detected in the peripheral blood from one HLA-DRB1*0301⁺ melanoma patient, verifying TRP-1₁₆₆₋₁₈₀ as a target epitope of spontaneous tumor-induced T cell responses. From two predicted potential HLA-DRB1*0401 epitopes, TRP-1₄₁₅₋₄₂₇ and TRP-2₂₈₈₋₃₀₂, the TRP-2 peptide exhibited strong, the TRP-1 peptide very strong HLA-DRB1*0401 binding capacity. Importantly in vivo sensitized T cells specific for TRP-1₄₁₅₋₄₂₇ could also be detected in the peripheral blood from one HLA-DRB1*0401⁺ melanoma patient, verifying TRP-1₄₁₅₋₄₂₇ as a target epitope of spontaneous tumor-induced T cell responses. Three sequences, derived from TRP-1 and TRP-2, respectively, were potential HLA-DQ8 epitopes. These peptides were directly used to immunize HLA-DQ8 transgenic mice. The peptide TRP-2₂₄₂₋₂₅₆ was clearly immunogenic in HLA-DQ8tg mice. HLA-DQ8 and HLA-DQ2 molecules have been demonstrated to present a largely overlapping peptide repertoire (Sidney et al., J. Immunol. 169 (2002), 5098). Interestingly, in vivo sensitized T cells specifically recognizing TRP-2₂₄₂₋₂₅₆ could be detected in an HLA-DQ2⁺ melanoma patient, indicating that TRP-2₂₄₂₋₂₅₆ is a target of spontaneous tumor-induced T cell responses. Thus, the application of the peptides of the present invention can improve the efficiency of immunotherapies of tumors, e.g., melanomas, since:
(a) the peptides of the invention specifically activate Th of the immune system of a patient and the activated Th support activation of specific CTL. The interaction of activated tumor antigen-specific Th and CTL is the prerequisite for an efficient adaptive immune response against the tumor resulting in the destruction of malignant cells;
(b) the peptides of the invention can be used for peptide-based vaccinations (e.g. peptide, peptide-loaded DCs) of tumor patients;
(c) the peptides of the invention are useful for the in vitro generation of autologous antigen-specific Th which can be applied to a patient for adoptive T cell therapy; and
(d) polynucleotides as (recombinant) RNA, (recombinant) DNA and recombinant viruses encoding the peptides of the invention can be used for the treatment of tumors.

Moreover, the peptides of the invention are useful for immune monitoring, i.e., for determining the presence of activated epitope-specific Th cells circulating in the blood of tumor patients and, thus, whether there is a spontaneous Th immune response against the tumor or a Th immune response induced by the vaccination.

### Brief description of the drawings

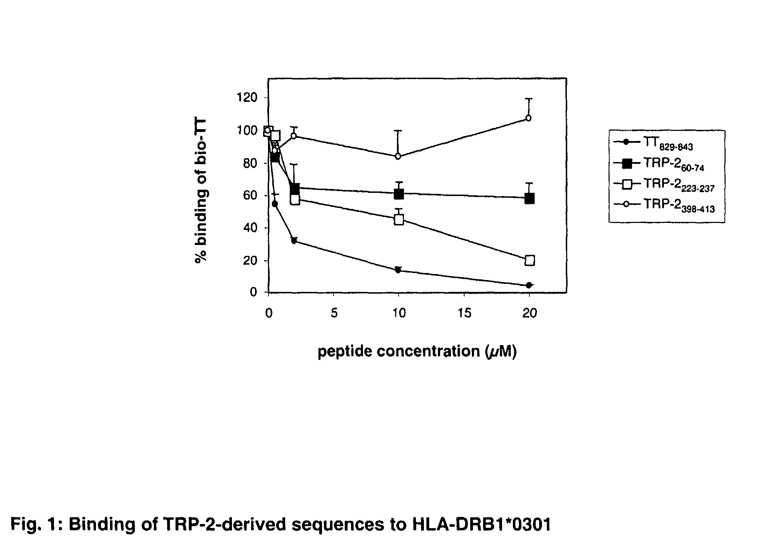

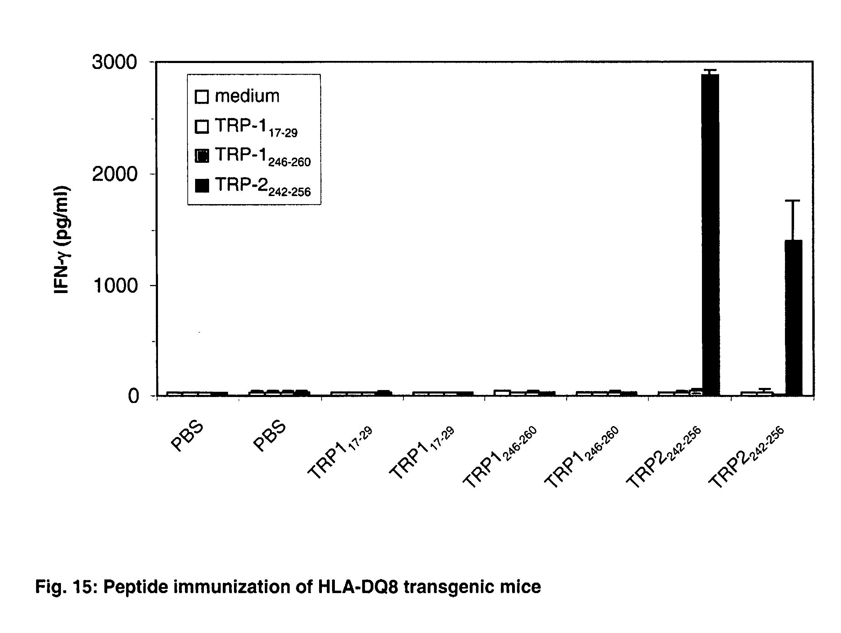

Figure 1: Binding of TRP-2-derived sequences to HLA-DRB1*0301
   T2.DR3 cells were pulsed with N-terminally biotinylated Tetanus Toxin peptide (10 µM TT₈₂₉₋₈₄₃; reporter peptide) for 180 min. After 30 min the indicated amounts of the competitor peptides TT, TRP-2₆₀₋₇₄ , TRP-2₂₂₃₋₂₃₇ and TRP-2₃₉₈₋₄₁₃, respectively, were added. Reporter peptide loading obtained after 180 min was quantified in a sandwhich immunoassay based on a time-resolved Europium fluorescence.

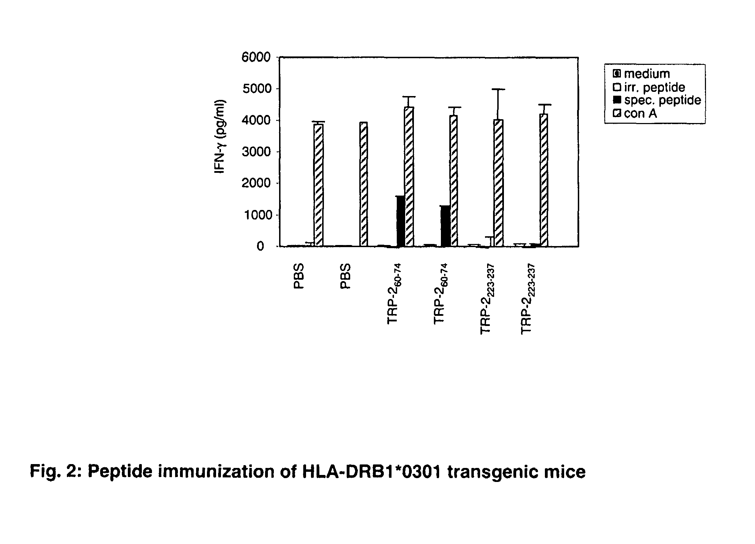

Figure 2: Peptide immunization of HLA-DRB1*0301 transgenic mice
   HLA-DRB1*0301 transgenic mice were immunized into hind foot pads with peptides TRP-2₆₀₋₇₄ or TRP2₂₂₃₋₂₃₇ emulsified in CFA. Control mice were treated with PBS-CFA. Ten days after immunization lymph node cells were harvested and analysed for their specificity. Therefore cells were incubated with medium, an irrelevant peptide (not used for immunization), the specific peptide used for immunization and concanavalin A (ConA; positive control that mediates unspecific T cell stimulation), respectively, for 48 hours. Culture supernatants were then analysed for the presence of IFN-γ secreted by stimulated T cells. All determinations were performed in triplicates. Data obtained are presented separately for each mouse. Peptide TRP-2₆₀₋₇₄ is clearly immunogenic in HLA-DR3tg mice whereas TRP2₂₂₃₋₂₃₇ reactive T cells were not detectable.

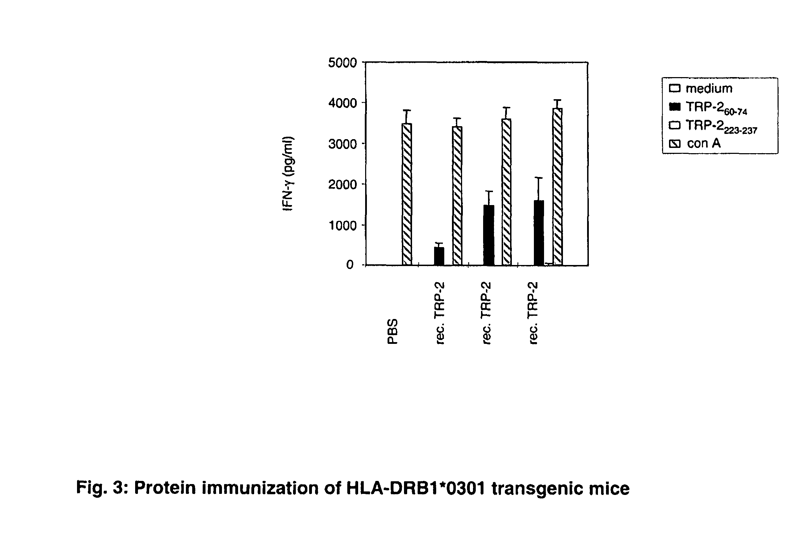

Figure 3: Protein immunization of HLA-DRB1*0301 transgenic mice
   HLA-DRB1*0301-transgenic mice were immunized with recombinant TRP-2 protein emulsified in CFA into hind foot pads (n=3). A control mouse was treated with PBS-CFA. Ten days after immunization lymph node cells were harvested and analysed for their specificity. Therefore cells were incubated with medium, peptide TRP-2₆₀₋₇₄, peptide TRP2₂₂₃₋₂₃₇ and concanavalin A (ConA; positive control that mediates unspecific T cell stimulation), respectively, for 72 hours. Culture supernatants were then analysed for the presence of IFN-γ secreted by stimulated T cells. All determinations were performed in triplicates. Data obtained are presented separately for each mouse. T cells recognizing peptide TRP-2₆₀₋₇₄ can be detected in lymph node cell cultures from protein-immunized mice, indicating that this sequence is processed from the global TRP-2 antigen.

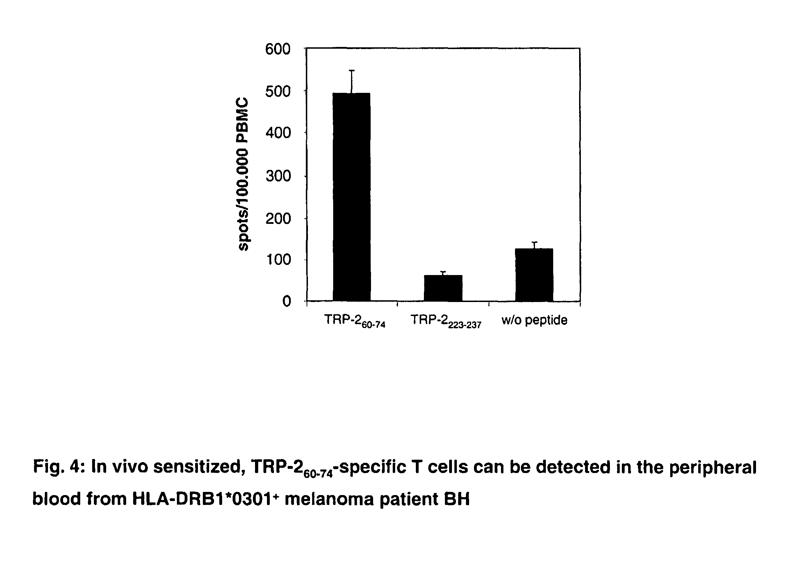

Fig. 4: In vivo sensitized, TRP-2₆₀₋₇₄-specific T cells can be detected in the peripheral blood from HLA-DRB1*0301⁺ melanoma patient BH
   PBMC from four HLA-DRB1*0301⁺ healthy donors and two HLA-DRB1*0301⁺ melanoma patients were incubated for 10 days without or with peptides (TRP-2_{60-74,} TRP-2₂₂₃₋₂₃₇). PBMC were then harvested and restimulated with the indicated peptides already used for primary stimulation whereas control cells were again left without peptide (w/o peptide). Subsequent IFN-γ secretion by T cells was measured by ELISPOT. All determinations were performed at least in triplicates. TRP-2₆₀₋₇₄-specific T cells can clearly be detected in the PBMC cultures from melanoma patient BH, whereas TRP-2₂₂₃₋₂₃₇-reactive T cells were not detectable.

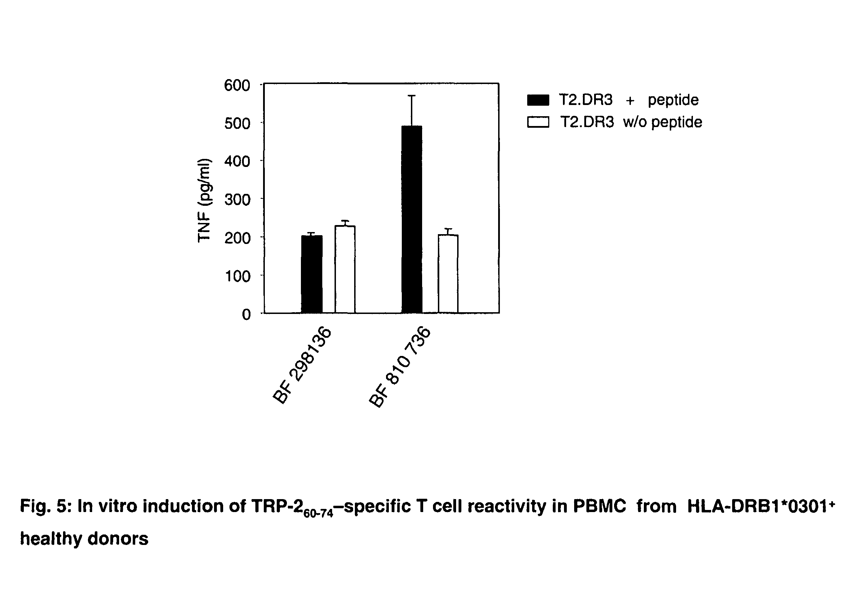

Fig. 5: In vitro induction of TRP-2₆₀₋₇₄-specific T cell reactivity in PBMC from HLA-DRB1*0301⁺ healthy donors
   PBMC from healthy donors were primarily stimulated with TRP-2₆₀₋₇₄-oaded DC. After the third restimulation, PBMC were analysed for peptide-specific reactivity. Therefore PBMC (1 x 10⁵) were incubated with T2.DR3 target cells (5 x 10⁴) in the presence or absence of TRP-2₆₀₋₇₄ peptide. Subsequent T cell-mediated TNF secretion was determined in an TNF bioassay. All determinations were performed at least in triplicates. TRP-2₆₀₋₇₄-specific T cells can clearly be detected in the PBMC cultures from donor BF 810736.

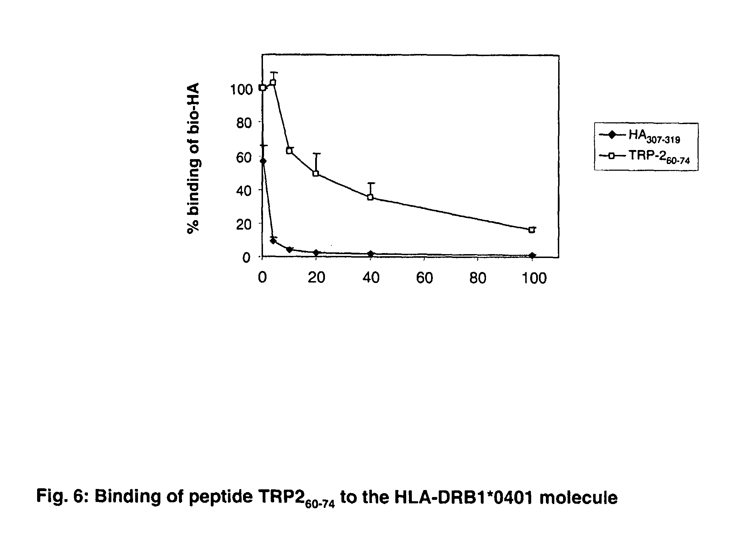

Figure 6: Binding of peptide TRP-2₆₀₋₇₄ to the HLA-DRB1*0401 molecule
   T2.DR4 cells were pulsed with N-terminally biotinylated influenza virus hemagglutinin peptide (10 µM HA₃₀₇₋₃₁₉; reporter peptide) for 180 min. After 30 min the indicated amounts of the competitor peptides, HA and TRP-2₆₀₋₇₄, respectively, were added. HA reporter peptide loading obtained after 180 min was quantified in a sandwhich immunoassay based on a time-resolved Europium fluorescence.

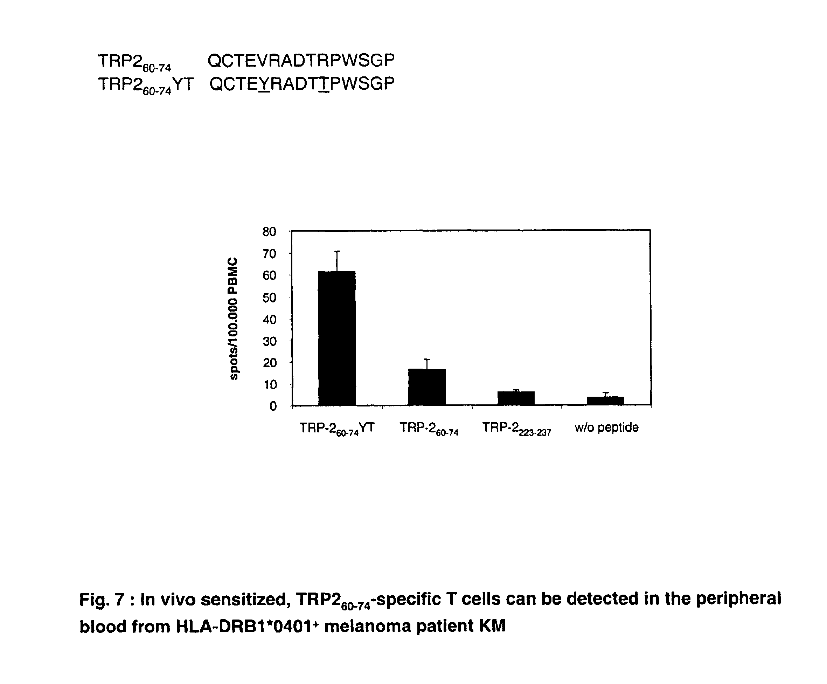

Fig. 7: In vivo sensitised, TRP-2 ₆₀₋₇₄ T cells can be detected in the peripheral blood from HLA-DRB1*0401⁺ melanoma patient KM
   PBMC from four HLA-DRB1*0401⁺ melanoma patients were incubated for 10 days without or with peptides (TRP-2₆₀₋₇₄, TRP-2₆₀₋₇₄YT, TRP-2₂₂₃₋₂₃₇), PBMC were then harvested and restimulated with the indicated peptides already used for primary stimulation whereas control cells were again left without peptide (w/o peptide). Subsequent IFN-γ secretion by T cells was measured by ELISPOT. All determinations were performed at least in triplicates. TRP-2₆₀₋₇₄-specific T cells can clearly be detected in the PBMC cultures from melanoma patient KM. T cell exhibited even stronger reactivity against the epitope variant TRP-2₆₀₋₇₄YT. There was no reactivity detectable against peptide TRP-2₂₂₃₋₂₃₇ which also exhibits strong HLA-DRB1*0401 binding capacity (data not shown).

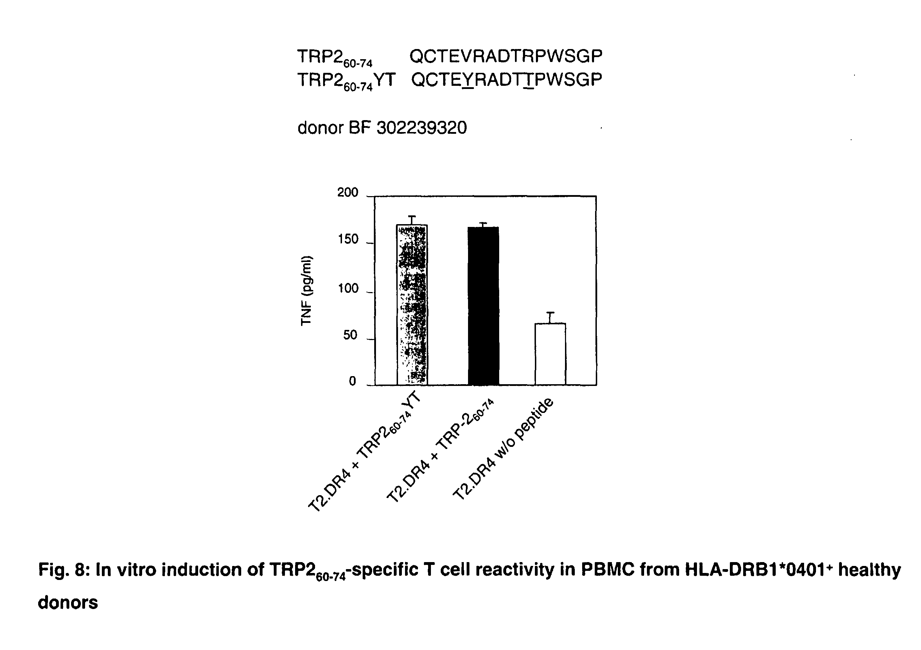

Figure 8: In vitro induction of TRP-2₆₀₋₇₄-specific T cell reactivity in PBMC from HLA-DRB1*0401⁺ healthy donors
   PBMC from healthy donor BF 302239320 were primarily stimulated with TRP-2₆₀₋₇₄YT-loaded DC. After the third restimulation PBMC were analysed for peptide-specific reactivity. Therefore PBMC (1 x 10⁵) were incubated with T2.DR4 target cells (5 x 10⁴) in the presence or absence of TRP-2₆₀₋₇₄YT and TRP2₆₀₋₇₄ peptide. Subsequent T cell-mediated TNF secretion was determined in a TNF bioassay. All determinations were performed in triplicates. T cells specific for the epitope variant TRP-2₆₀₋₇₄ YT could clearly be detected in the PBMC culture. These cells exhibited cross-reactivity against the natural peptide TRP-2₆₀₋₇₄.

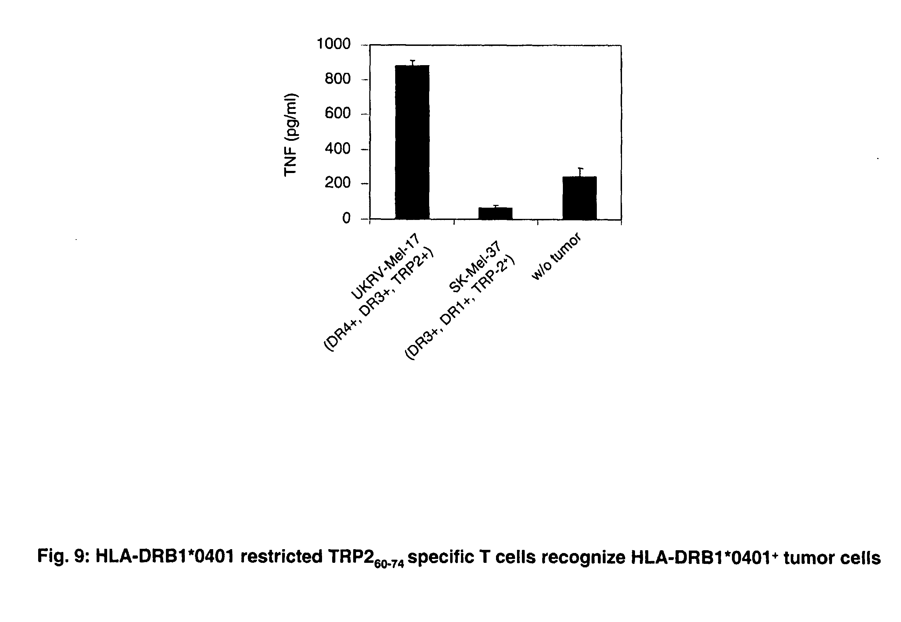

Figure 9: HLA-DRB1*0401 restricted TRP-2₆₀₋₇₄ specific T cells recognize HLA-DRB1*0401⁺ tumor cells
   Peptide-specific T cells from PBMC culture from donor BF 302239320 were analysed for their capability to recognize HLA-DRB1*0401⁺, TRP-2⁺ tumor cells. Therefore T cells were incubated with melanoma target cells (UKRV-Mel-17 [HLA-DRB1*0401⁺, TRP-2⁺], SK-Mel-37 [HLA-DRB1*0401⁻, TRP-2^{*}]) at a ratio of 10:1. Secretion of TNF by stimulated T cells was determined in a TNF secretion assay.

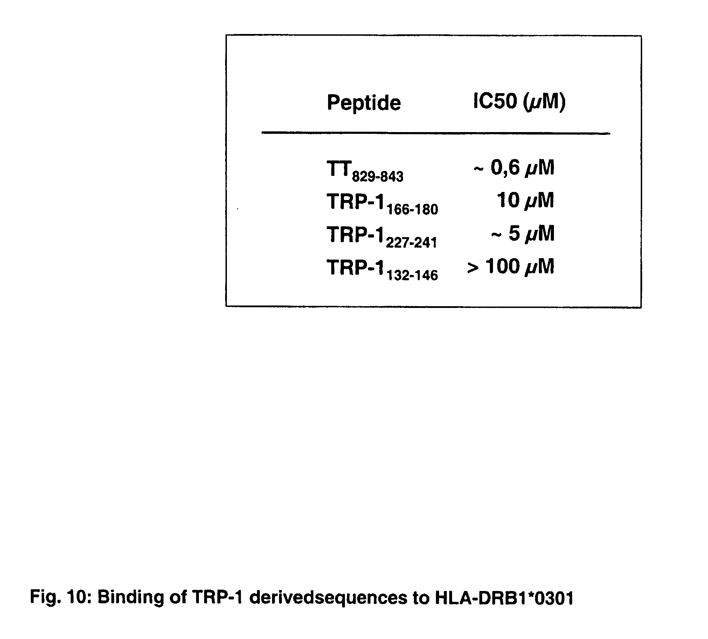

Figure 10: Binding of TRP-1 derived seauences to HLA-DRB1*0301
   T2.DR3 cells were pulsed with N-terminally biotinylated Tetanus Toxin peptide (10 µM TT₈₂₉₋₈₄₃ reporter peptide) for 180 min. After 30 min the indicated amounts of the competitor peptides TT, TRP-1₁₃₂₋₁₄₆, TRP-1₁₆₆₋₁₈₀ and TRP-1₂₂₁₋₂₄₁, respectively, were added. TT reporter peptide loading obtained after 180 min was quantified in a sandwich immunoassay based on a time-resolved Europium fluorescence. IC50 is the dose necessary to reduce TT reporter peptide binding to 50%.

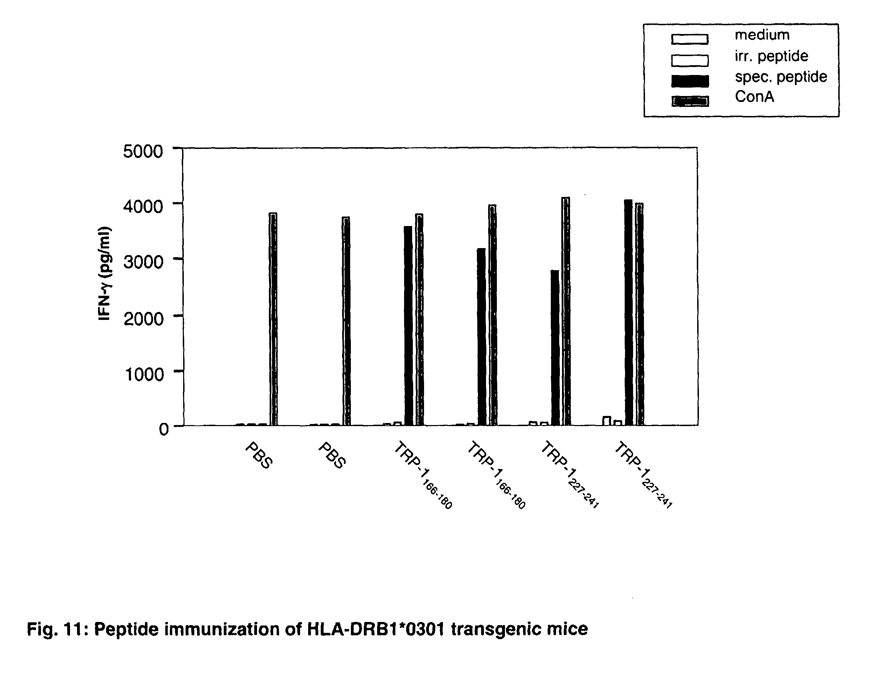

Figure 11: Peptide immunization of HLA-DRB1*0301 transgenic mice
   HLA-DRB1*0301 transgenic mice were immunized into hind foot pads with peptides TRP-1₁₆₆₋₁₈₀ or TRP1₂₂₇₋₂₄₁ emulsified in CFA. Control mice were treated with PBS-CFA. Ten days after immunization lymph node cells were harvested and analysed for their specificity. Therefore cells were either incubated with medium, with an irrelevant peptide (not used for immunization), with the specific peptide used for immunization and with concanavalin A (ConA; positive control that mediates unspecific stimulation of T cells), respectively, for 48 hours. Culture supernatants were then analysed for the presence of IFN-γ secreted by stimulated T cells. All determinations were performed in triplicates. Data obtained are presented separately for each mouse. Both peptides are clearly immunogenic in HLA-DR3tg mice.

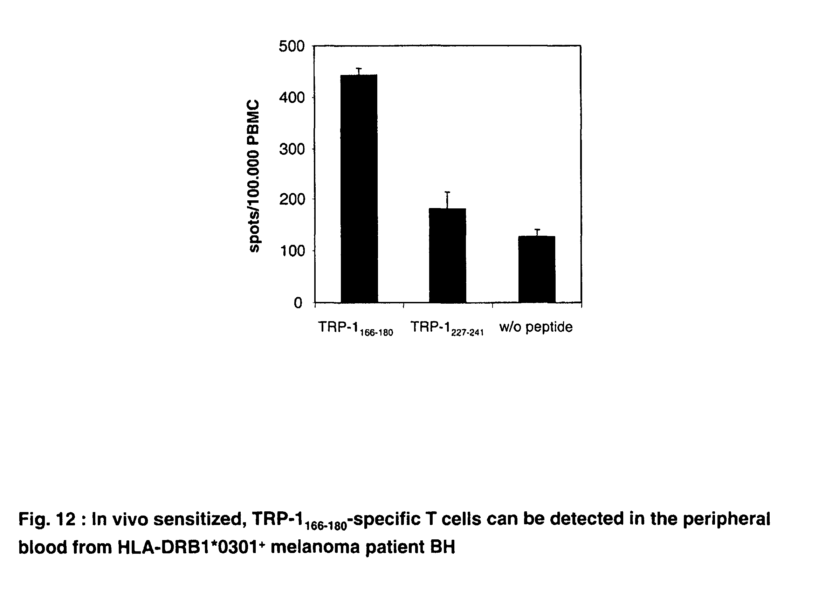

Figure 12: In vivo sensitised, TRP-1₁₆₆₋₁₈₀ T cells can be detected in the peripheral blood from HLA-DRB1*0301 melanoma patient BH
   PBMC from four HLA-DRB1*0301⁺ healthy donors and two HLA-DRB1*0301⁺ melanoma patients were incubated for 10 days without or with peptides (TRP-1₁₆₆₋₁₈₀, TRP-1₂₂₇₋₂₄₁). PBMC were then harvested and restimulated with the indicated peptides already used for primary stimulation whereas control cells were again left without peptide (w/o peptide). Subsequent IFN-γ secretion by T cells was measured by ELISPOT. All determinations were performed at least in triplicates. TRP-1₁₆₆₋₁₈₀-specific T cells can clearly be detected in the PBMC cultures from melanoma patient BH, whereas TRP-1₂₂₇₋₂₄₁-specific reactivity is not obvious.

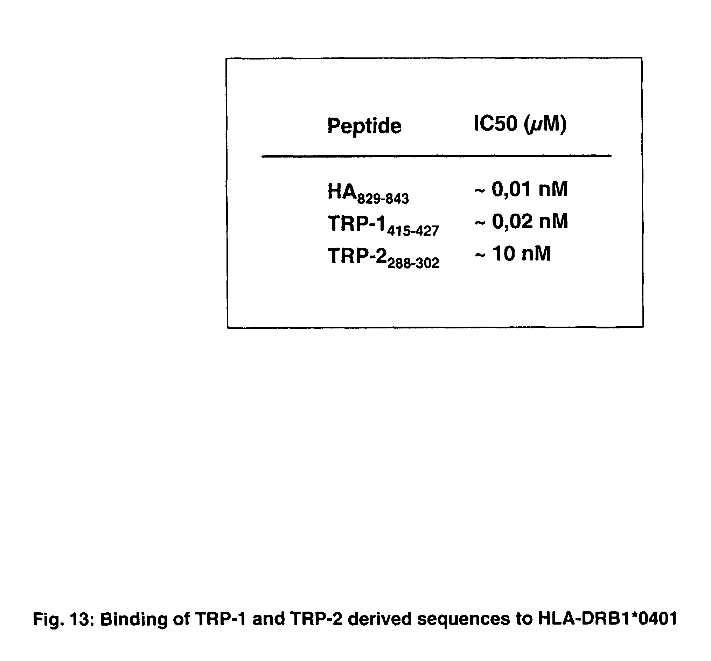

Figure 13: Binding of TRP-1 and TRP-2 derived sequences to the HLA-DRB1*0401 molecule
   T2.DR4 cells were pulsed with N-terminally biotinylated influenza virus hemagglutinin peptide (10 µM HA₃₀₇₋₃₁₉ ; reporter peptide) for 180 min. After 30 min the indicated amounts of the competitor peptides HA, TRP-1₄₁₅₋₄₂₇ and TRP-2₂₈₈₋₃₀₂, respectively, were added. HA reporter peptide loading obtained after 180 min was quantified in a sandwhich immunoassay based on a time-resolved Europium fluorescence. IC50 is the dose necessary to reduce TT reporter peptide binding to 50%.

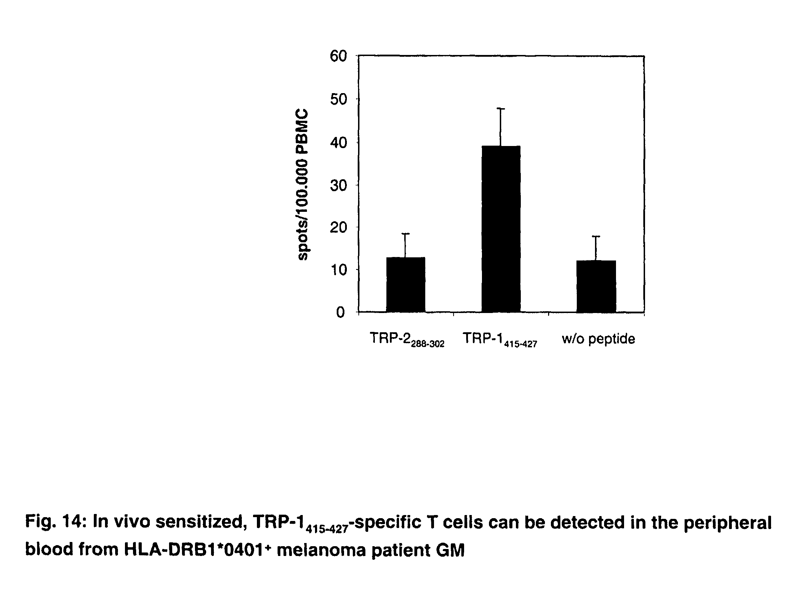

Figure 14: In vivo sensitized, TRP-1₄₁₅₋₄₂₇-specific T cells can be detected in the peripheral blood from HLA-DRB1*0401⁺ melanoma patient GM
   PBMC from four HLA-DRB1*0401⁺ melanoma patients were incubated for 10 days without or with peptides (TRP-1₄₁₅₋₄₂₇, TRP-2₂₈₈₋₃₀₂). PBMC were then harvested and restimulated with the indicated peptides already used for primary stimulation whereas control cells were again left without peptide (w/o peptide). Subsequent IFN-γ secretion by T cells was measured by ELISPOT. All determinations were performed at least in triplicates. TRP-2₄₁₅₋₄₂₇-specific T cells can clearly be detected in the PBMC cultures from melanoma patient GM. There was no reactivity detectable against TRP-2₂₈₈₋₃₀₂
Figure 15: Peptide immunization of HLA-DQ8 transgenic mice
   HLA-DQ8 transgenic mice were immunized into hind foot pads with peptides TRP-1₁₇₋₂₉, TRP-1₂₄₆₋₂₆₀ or TRP-2₂₄₂₋₂₅₆ emulsified in CFA. Control mice were treated with PBS-CFA. Ten days after immunization lymph node cells were harvested and analysed for their specificity. Therefore cells were incubated with medium, with irrelevant peptides (not used for immunization) and with the specific peptide used for immunization, respectively, for 48 hours. Culture supernatants were then analysed for the presence of IFN-γ secreted by stimulated T cells. All determinations were performed in triplicates. Data obtained are presented separately for each mouse. T cells reactive for peptide TRP-2₂₄₂₋₂₅₆ can clearly be detected after immunization.

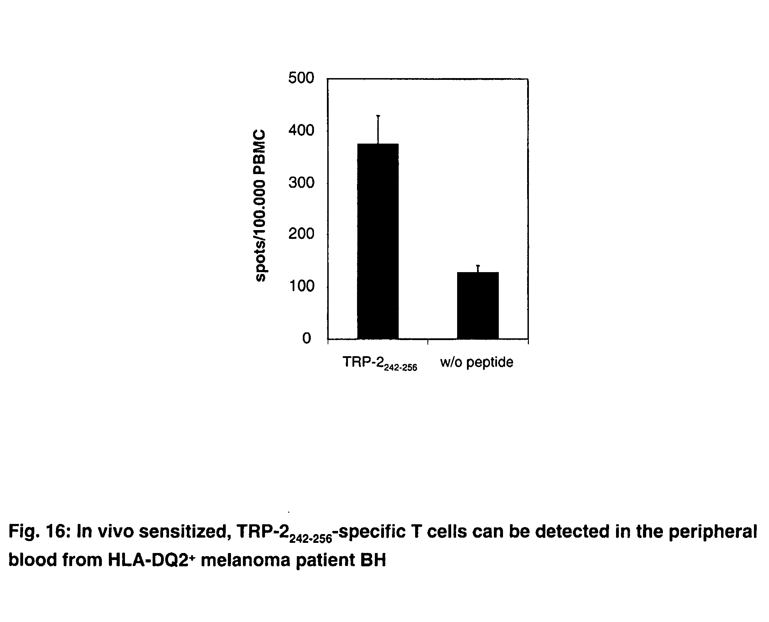

Figure 16: In vivo sensitized, TRP-2₂₄₂₋₂₅₆-specific T cells can be detected in the peripheral blood from HLA-DO2⁺ melanoma patient BH
   PBMC from melanoma patient BH were incubated for 10 days without or with peptide TRP-2₂₄₂₋₂₅₆. PBMC were then harvested and restimulated with the peptide already used for primary stimulation whereas control cells were again left without peptide (w/o peptide). Subsequent IFN-γ secretion by T cells was measured by ELISPOT. All determinations were performed at least in triplicates. TRP-2₂₄₂₋₂₅₆-reactive T cells can clearly be detected in the PBMC cultures from melanoma patient BH. This supports the finding that a lot of HLA-DQ8 binding peptides can also function as HLA-DQ2 presented epitopes.

Accordingly, the present invention relates to TRP-2- and TRP-1-derived peptides capable of activating CD4⁺ T helper cells (Th) against a TRP-2⁺ and/or TRP-1⁺ tumor, wherein said peptides comprise
(a) the amino acid sequences QCTEVRADTRPWSGP (human TRP-2₆₀₋₇₄), QCTEYRADTTPWSGP (TRP-2₆₀₋₇₄YT, epitope variant of human TRP-2₆₀₋₇₄), LPYWNFATGRNECDV (human TRP-2₂₄₂₋₂₅₆), SEEILGPDGNTPQFE (human TRP-1₁₆₆₋₁₈₀), LRRYNADISTFPL (human TRP-1₄₁₅₋₄₂₇)
(b) an amino acid sequence which differs from an amino acid sequence from (a) by one, two, three, four or more amino acid substitutions (e.g. QCTEYRADTTPWSGP (TRP-2₆₀₋₇₄ YT, epitope variant of human TRP-2₆₀₋₇₄)
(c) a peptide having an amino acid sequence which shows at least 50% identity to the amino acid sequence of the peptide of (a) or (b);
(d) a peptide having an amino acid sequence which shows at least 70% identity to the amino acid sequence of the peptide of (a) or (b);
(e) a peptide having an amino acid sequence which shows at least 90% identity to the amino acid sequence of the peptide of (a) or (b); or
(f) a peptide which is a fragment of the peptide of (a), (b), (c),(d) or (e);
wherein the peptides of (b), (c), (d), (e) or (f) are still capable of activating CD4⁺ T helper cells (Th) against a TRP-2⁺ and/or TRP-1⁺ tumor.

The term "amino acid substitutions" involves replacement of any of the amino acids constituting the peptides of the invention by any of the natural 20 amino acids or chemically variants of the natural amino acids. The term "amino acid substitutions" preferably means conservative amino acid substitutions.

The term "conservative amino acid substitutions" involves replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Preferably, the peptides of the invention are characterized by not more than 8 aa substitutions, more preferably by not more than 6 aa substitutions and, even more preferably, by not more than 4 aa substitutions.

The peptide variants preferably exhibit at least about 50% identity, more preferably at least about 70 or 80% identity and most preferably at least about 90% identity to a TRP-2 or TRP-1 peptide with the specific amino acid sequences disclosed herein. Two polypeptide sequences are said to be "identical" if the sequence of amino acids in the two sequences is the same when aligned for maximum correspondence as described below. Comparisons between two sequences are typically performed by comparing the sequences over a comparison window to identify and compare local regions of sequence similarity. Optimal alignment of sequences for comparison may be conducted using the Megalign program in the Lasergene suite of bioinformatics Software (DNASTAR, Inc., Madison, W1), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* 183, Academic Press, Inc., San Diego, CA; Higgins, D.G. and Sharp, P.M. (1989) CABIOS 5:151-153; Myers, E.W. and Muller W. (1988) *CABIOS* 4:11-17; Robinson, 20 E.D. (1971) *Comb. Theor* 11:105; Santou, N. Nes, M. (1987) *Mol. Biol. Evol.* 4:406425; Sneath, P.H.A. and Sokal, R.R. (1973) *Numerical Taxonomy - the Principles and Practice of Numerical Taxonomy,* Freeman Press, San Francisco, CA; Wilbur, W.J. and Lipman, D.J. (1983) *Proc. Natl. Acad., Sci.* USA 80:726-730.

For the generation of peptides showing a particular degree of identity to a TRP-1 or TRP-2 peptide, e.g., genetic engineering can be used to introduce amino acid changes at specific positions of a cloned DNA sequence to identify regions critical for peptide function. For example, site directed mutagenesis or alanine-scanning mutagenesis (introduction of single alanine mutations at every residue in the molecule) can be used. (Cunningham and Wells, Science 244 (1989), 1081-1085). Alternatively, peptides showing a particular degree of identity to the TRP-2 or TRP-1 peptides can be chemically synthesized by standard procedures as mentioned in Example 1. The resulting mutant molecules can then be tested for immunological activity using the assays of Example 1.

In the fragment of the peptides of the invention at least 3 contiguous aa, preferably at least 5 contiguous aa, more preferably at least contiguous 7 aa and even more preferably at least 9 contiguous aa of the amino acid sequence are left. The fragments are still capable of activating CD4⁺ T helper cells (Th) against a TRP-2⁺ and/or TRP-1⁺ tumor. For evaluating whether a particular fragment (or derivative of a TRP-2 or TRP-1 peptide characterized by substitutions of amino acids) is still capable of activating CD4⁺ T helper cells (Th) against a TRP-2⁺ and/or TRP-1⁺ tumor an assay of Example 1 can be used.

TRP-2⁺ and/or TRP-1⁺ tumors comprise neuroectoderm-derived tumors with malignant melanoma and glioblastoma being preferred.

The present invention also relates to a fusion protein comprising a peptide of the invention. The fusion protein comprises multiple peptides as described herein or comprises at least one peptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the peptide or to enable the peptide to be targeted to desired intracellular compartments. Still further fusion partners include affinity tags, which facilitate purification of the peptide. Fusion proteins may generally be prepared using standard techniques, including chemical conjugation. Preferably, a fusion protein is expressed as a recombinant protein, allowing the production of increased levels, relative to a non-fused peptide in an expression system. Briefly, DNA sequences encoding the (poly)peptide components may be assembled separately, and ligated into an appropriate expression vector. The 3'-end of the DNA sequence encoding one peptide component is ligated, with or without a peptide linker, to the 5'-end of a DNA sequence encoding the second (poly)peptide component so that the reading frames of the sequences are in phase. This permits translation into a single fusion protein that retains the biological activity of both component (poly)peptides. A peptide linker sequence may be employed to separate the first and the second components by a distance sufficient to ensure that each (poly)peptide folds into its secondary and tertiary structures. Such a peptide linker sequence is incorporated into the fusion protein using standard techniques well known in the art. Suitable peptide linker sequences may be chosen based an the following factors: (1) their ability to adopt a flexible extended conformation; (2) their inability to adopt a secondary structure that could interact with functional epitopes in the first and second (poly)peptides; and (3) the lack of hydrophobic or charged residues that might react with the (poly)peptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., *Gene* 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length.

Fusion proteins are also provided that comprise a peptide of the present invention together with an unrelated immunogenic protein.

The present invention also relates to polynucleotides encoding a peptide or multiple peptides of the invention, preferably fused to secretory leader sequences, and to polypeptides encoding a peptide or multiple peptides of the invention fused to a foreign protein, preferably a secreted protein, as well as expression vectors which are capable of expressing the peptide of the invention and which can be used for gene therapy. Preferred recombinant vectors useful for gene therapy are viral vectors, e.g. adenovirus, herpes virus, vaccinia virus, modified vaccinia virus Ankara (MVA), influenza virus, lentivirus or retrovirus. More preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the viral vectors can be modified in such a way that they become target specific. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, the polynucleotide encoding a peptide of the invention can also be operably linked to a tissue specific promoter and used for gene therapy. Such promoters are well known to those skilled in the art (see e.g. Zimmermann et al, (1994) Neuron 12, 11-24; Vidal et al., (1990) EMBO J. 9, 833-840; Mayford et al., (1995), Cell 81, 891-904; Pinkert et al., (1987) Genes & Dev. 1, 268-76).

The present invention also relates to host cells transformed or transfected with an expression vector of the invention. Suitable host cells include prokaryotes, yeast and higher eukaryotic cells. Preferably, the host cells employed are E. coli, yeast or a mammalian cell line such as COS or CHO or primary mammalian cells such as macrophages or dentritic cells. Supernatants from suitable host/vector systems which secrete a recombinant peptide or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant (poly)peptide.

The present invention also relates to antibodies specifically binding to a peptide of the invention or to a complex consicting of any HLA molecule and a peptide of the invention. The term "antibody", preferably, relates to distinct monoclonal antibody preparations. Monoclonal antibodies are made against a peptide of the invention or a fragment thereof or against a peptide-HLA complex as an antigen by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to proteins. Fab and F(ab')2 fragments lack the Fc fragment of intact antibodies, clear more rapidly from the circulation, and may have less nonspecific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies. The target cellular component in biological fluids or tissues, i.e. TRP-2 or a TRP-2 encoding mRNA, may be detected directly in situ or it may be isolated from other cell components by common methods known to those skilled in the art before contacting with a probe. Detection methods include Northern blot analysis, RNase protection, in situ methods, PCR, LCR, immunoassays and other detection assays that are known to those skilled in the art.

In a further embodiment, the present invention relates to a pharmaceutical composition containing a peptide, polynucleotide, an expression vector or antibody of the invention. For administration the compounds of the pharmaceutical composition are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions, nanoparticles etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the particular nature and location of the TRP-2⁺ and/or TRP-1⁺ tumor and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the tumor, general health and other drugs being administered concurrently.

The present invention also relates to various therapeutic uses of the compounds of the present invention. A preferred use is the treatment of a TRP-2⁺ and/or TRP-1⁺ tumor, preferably said treatment is stimulation of an immune response.

Preferred tumors that can be treated/diagnosed by the compounds of the present invention are melanoma, glioblastoma and other tumors of neuroectodermal origin.

The present invention also relates to a diagnostic composition containing a peptide, fusion protein or antibody of the invention as well as diagnostic kits comprising a peptide, fusion protein and/or antibody of the invention and a detection agent. Such kits typically comprise two or more components necessary for performing a diagnostic assay. Components may be compounds, reagents, containers and/or equipment. For example, one container within a kit may contain a monoclonal antibody or fragment thereof that specifically binds to a TRP-2⁺ and/or TRP-1⁺ tumor protein. Such antibodies or fragments may be provided attached to a support material. The solid support may be any material known to those of ordinary skill in the art to which the tumor protein may be attached. For example, the solid support may be a test well in a microtiter plate or a nitrocellulose or other suitable membranes. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent (e.g., peptide or antibody of the invention) ranging from about 10 ng to about 10 µg is sufficient to immobilize an adequate amount of binding agent. Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner *(See, e.g.,* Pierce Immunotechnology Catalog and Handbook, 1991, at A12-A13).

One or more additional containers may enclose elements, such as reagents or buffers, to be used in the assay. Such kits may also, or alternatively, contain a detection reagent that contains a reporter group suitable for direct or indirect detection of, e.g., antibody binding. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Preferably, said detection agent is a second antibody capable of binding to a different site on the polypeptide containing a reporter group. The amount of detection reagent that remains bound to the solid support is then determined using a method appropriate for the specific reporter group.

Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent.

Finally, the detection reagent might be diagnostic agent that is detectable upon imaging, e.g., magnetic resonance imaging (MRI), PET etc.. In a preferred embodiment of the present invention, said diagnostic agent is a paramagnetic ion, radioactive ion or a fluorogenic ion. Specifically, the diagnostic agents utilized in embodiments of the invention include: chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), erbium (III), iodine¹²³, technetium^{99m}, indium¹¹¹, rhenium¹⁸⁸, rhenium¹⁸⁶, copper⁶⁷, iodine¹³¹, yttrium⁹⁰, iodine¹²⁵, astatine²¹¹, and gallium⁶⁷.

The present invention also relates to various uses of the diagnostic composition (kit) of the present invention. Preferred uses are:
(a) monitoring an (induced) Th immune response against a TRP-2⁺ and/or TRP-1⁺ tumor, preferably by using a peptide or fusion protein of the present invention; and
(b) detecting the presence of a TRP-2⁺ and/or TRP-1⁺ tumor in a patient, preferably by using an antibody of the present invention.

The present invention is explained by the following examples.

### Example 1

### General methods

### (A) HLA-DR3 and HLA-DQ8 Transgenic Mice

HLA-DRB1*0301 (HLA-DR3)and HLA-DQ8 transgenic mice lacking expression of endogenous class II molecules (Kong et al., J. Exp. Med. 184 (1996), 1167; Flynn et al., Hum. Immunol. 63 (2002), 63, 301) were kindly provided by Dr. C.S. David, Mayo Clinic, Rochester, MN. Mice were bred in isolators or individually ventilated cages and transgene expression was tested by flow cytometry of PBLs stained with a FITC-conjugated HLA-DR-specific monoclonal antibody (Becton Dickinson, Heidelberg, Germany).

### (B) Culture Media

Murine lymph node cells were cultured in DMEM (Sigma) containing 10% FCS (Biochrom), 2 mM glutamine, penicillin/streptomycin and 5x10⁻⁵ M 2-mercaptoethanol.

### (C) Expression and purification of recombinant TRP2 protein

The cDNA of TRP-2 (bases 69-1398) (forward primer: 5'-CAGTTCCCCCGAGTCTGCATG-3'; reverse primer: 5'-AGTTTCTTCAACTGAAACTGGCAG-3') was cloned in the expression vector pTrcHis-TOPO (Invitrogen), in which the TRP-2-gene is under control of an IPTG-inducible trc-promotor. The expression plasmid was transformed into *Escherichia coli* BL21-CodonPlus-RIL and bacteria were grown at 37°C in SOB-Medium (Invitrogen). Expression of the X-press-tagged protein was induced by the addition of IPTG to a final concentration of 1 mM. After 3 h of induction, bacteria were pelleted and lysed in buffer A (6 M GuHCl, 100 mM NaH₂PO₄, 10 mM Tris-Cl, pH 8,0). The lysate was stirred for 1 h before centrifugated to pellet the cellular debris. As determined by SDS-PAGE and Western Blot analysis, employing an anti-X-press mAb (Invitrogen), the recombinant protein was present in the cleared lysate. A suspension of 1 ml Ni-NTA-Agarose Superflow was equilibrated in buffer A and the solubilized protein was allowed to bind by tumbling for 1 h. The beads were poured in a FPLC-column, which was sequentially washed with buffer A and buffer B (8 M Urea, 100 mM NaH₂PO₄, 10 mM Tris-Cl, pH 8,0). The bound protein was eluted with buffer E (8 M Urea, 100 mM NaH₂PO₄, 10 mM Tris-Cl, pH 4,5). The eluted proteins were reactive with the anti-X-press antibody, and purity was >50% as determined by SDS-PAGE. The proteins were dialysed twice against 0,1 x PBS. The precipitated protein was washed twice with PBS and resuspended in CFA for immunization experiments.

### (D) Synthetic Peptides

The following peptides were synthesized by Fmoc chemistry and analyzed by HPLC at the Division of Peptide Synthesis of the DKFZ, Heidelberg: MQYIKANSKFIGITE (TT₈₂₉₋₈₄₃), PKYVKQNTLKLAT (HA₃₀₇₋₃₁₉), QCTEVRADTRPWSGP (TRP-2₆₀₋₇₄), QCTEYRADTTPWSGP (TRP-2₆₀₋₇₄YT, epitope variant of human TRP-2₆₀₋₇₄), HLLCLERDLQRLIGN (TRP-2₂₂₃₋₂₃₇), LPYWNFATGRNECDV (TRP-₂₄₂₋₂₅₆), SLDDYNHLVTLCNGT (TRP-2₂₈₈₋₃₀₂); FTDAIFDEWMKRFNPP (TRP-2₃₉₈₋₄₁₃), NLLDLSKEEKNHFVR (TRP-1₁₃₂₋₁₄₆), SEEILGPDGNTPQFE (TRP-1₁₆₆₋₁₈₀), HLLRLEKDMQEMLQE (TRP-1₂₂₇₋₂₄₁), LRRYNADISTFPL (TRP-1₄₁₅₋₄₂₇)·

### (E) HLA-DR-Binding Assay

The relative binding affinity of the TRP-2- and TRP-1-derived sequences was determined as follows: T2.DR3 or T2.DR4 cells were loaded with biotinylated reporter peptides (TT₈₂₉₋₈₄₃ for T2.DR3, HA₃₀₇₋₃₁₉ for T2.DR4). After 30 min competitor peptides were added. Cells were rotated for 180 min, washed and lysed with 20 mM Tris pH 7.4, 5 mM MgCl₂, 1 % NP-40 supplemented with protease inhibitors. Quantitation of HLA-DR-peptide complexes was performed by sandwich immunoassay. The lysate was diluted 10-fold in phosphate buffered saline containing 0,05% Tween-20 and 1% bovine serum albumin and incubated in an L243-coated microtiter plate (Nunc) for 3 h. Plates were developed by incubation for 45 min with 0,1µg/ml streptavidin-Europium (Perkin Elmer) according to the manufacturer's protocol and time-resolved fluorescence was quantified using the VIC-TOR multilabel counter (Perkin Elmer).

### (F) HLA-Typing of blood donors

HLA-DR specificity of PBMC from healthy donors and melanoma patients was determined by high-resolution PCR typing: healthy donors BF 298136 (HLA-A1, -A2, -B8, -B62, -DRB1*0301, - DRB1*1301), BF 810736 (HLA-A1, -A24, -B8, -B52, -DRB1*0301, - DRB1*1502), BF 302239320 (HLA-A3, -B51, -B56, -DRB1*0401, - DRB1*1303); melanoma patients BH (A24, A33, B35, B58, DR3, DR8, DQ4, DQ2) KM (A2, A24, B15, B57, DQ3, DQ5, DR1, DR4), GM (A2, A28, B7, B56, DRB1*04, DRB1*1201).

### (G) Immunizations and Analysis of T cell responses

Mice were injected with 100 µg synthetic peptide or with 10-15 µg recombinant TRP-2 emulsified 1:1 in complete Freund's adjuvant (CFA) (Sigma) into hind foot pads. Ten to twelve days later, single cell suspensions prepared from draining lymph nodes (popliteal, inguinal and lumbar) were tested for peptide specificity in 96 well flat bottom microtiter plates seeding 4x10⁵ lymph node cells and adding 10 µg peptide or 1 µg concanavalin A (unspecific T cell stimulation) per well. After 72 h, the amount of released IFN-γ was determined by testing 100 µl supernatant removed from each well by ELISA (Becton-Dickinson Biosciences, Heidelberg, Germany) according to the manufacturer's instructions.

### (H) In vitro induction of peptide specificity in mixed PBMC/dentritic cell cultures

Peripheral blood mononuclear cells (PBMC) were isolated from peripheral blood of healthy donors by Ficoll Hypaque gradient and frozen until use. Thawed PBMC were either used as a source of CD4⁺ T cells or as a source of monocytes for the generation of dendritic cells (DC) following a routine protocol (Sun et al., Int. J. Cancer 87 (2000), 399). Briefly, plastic-adherent monocytes were incubated in X-VIVO-15 (Boehringer Ingelheim, Heidelberg, Germany) supplemented with 10% human serum and IL-4 (800 U/ml) and GM-CSF (1000 U/ml) over 5 days to obtain immature DC. On day 6 the cytokines IL-1β (10 ng/ml), IL-6 (1000 U/ml), PGE (1 µg/ml) and TNF-α (10 ng/ml) were added to the cultures to induce DC maturation. In parallel, peptide (10 µg/ml) was added. After 48 hours, DC were harvested and used to stimulated 1 x 10⁶ PBMC at a ratio of 1:20 (DC:PMBC). Twenty-four hours later IL-2 (20 U/ml) was added to the cultures. IL-2 (5 U/ml) was added every other day. Responder T cells were restimulated at a 14-day intervals with mitomycin-inactivated, autologous PBMC loaded with peptide (10 µg/ml). T cell cultures were screened for peptide reactivity after the first restimulation.

### (I) Detection of memory T cell responses by ELISPOT

Frozen peripheral blood mononuclear cells (PBMC) from normal donors and melanoma patients were thawed and seeded at 2 x 10⁶ cells/ml per well of a 24 well plate in IMDM supplemented with 10% FCS and 2mM glutamine. Peptide was added at a concentration of 10 µg/ml, control cells were left without peptide. After 10 days cells were harvested and screened for peptide-reactivity.

### (J) ELISpot Assay

To detect peptide-reactive T cells in the peripheral blood of healthy donors and melanoma patients, IFN-γ ELISPOT assays was performed. Therefore, 96-well micro filtration plates (Millipore) were coated with 5 µg/ml of anti-human IFN-γ mAb (1-D1K; Mabtech) in PBS overnight at 4°C. Unbound antibody was removed by washing with PBS. After blocking the plates with RPMI 1640/10% human serum (1 h at 37°C), 10⁵ PBMC/well were seeded. Synthetic peptides (stocks at 50 mg/ml DMSO) were then added to appropriate wells at a final concentration of 10 µg/ml. Controls were not incubated with peptide. Culture medium was added at a final volume of 200 µl/well. Plates were incubated at 37°C in 5% CO₂ for 16 h. After incubation, plates were washed to remove cells. Captured cytokinewas detected at sites of its secretion by incubation for 2 hwith biotinylated mAb anti-human IFN-γ (7-B6-1; Mabtech) at 1 µg/ml in PBS/0.5% BSA. Plates were then washed and avidin-peroxidase complex (Vectastain Elite Kit; Vector Laboratories) was added for 1 h. Unbound complex was removed by washings and then AEC substrate (Sigma) was added and incubated for 10 min. All determinations were performed in triplicates. Spots were imaged using the Biosys Bioreader 3000. The data are represented as mean IFN-γ spots per 100.000 PBMC.

### (K) TNF Bioassay

To determine TNF secretion by stimulated T cells, supernatants from T cell-target cell cultures were harvested 20 hours after coincubation. TNF content was estimated by measuring lysis of WEHI 164 clone 13 in a MTT colorimetric assay as described previously (Sun et al., Int. J. Cancer 87 (2000), 399).

### Example 2

### Peptide selection and HLA-binding studies

The screening of the human TRP-2 sequence for potential HLA-DRB1*0301 restricted epitopes by computer algorithms led to the selection of three peptide sequences as candidate epitopes: TRP-2₆₀₋₇₄, TRP-2₂₂₃₋₂₃₇ and TRP-2₃₉₈₋₄₁₃. These sequences are characterized by an aspartat residue at position 4 of the 9 mer core sequence, a feature of most HLA-DRB1*0301 binding peptides. The capacity of these sequences to bind to the HLA-class II molecule was analyzed in a competitive binding assay employing a Tetanus Toxin (TT₈₂₉₋₈₄₃) peptide as an already known control HLA-DRB1*0301 binder (Figure 1). The sequences TRP-2₃₉₈₋₄₁₃ did not exhibit any binding capacity for the HLA-class II molecule, even at high peptide concentrations. In contrast, the 15mers TRP-2₆₀₋₇₄ and TRP-2₂₂₃₋₂₃₇ bound to HLA-DRB1*0301. Both sequences have comparable binding affinities at low peptide concentrations but this dramatically changes at higher concentrations. Whereas TRP-2₂₂₃₋₂₃₇ peptide exhibits an IC₅₀ of 7,5 µM this value could not be determined for peptide TRP-2₆₀₋₇₄ due to its biphasic binding properties.

### Example 3

### Analysis of peptide immunogenicity in HLA-DRB1*0301 transgenic mice

Based on their HLA-DR binding capacity, the 15mers TRP-2₆₀₋₇₄ and TRP-2₂₂₃₋₂₃₇ were selected for peptide immunization of HLA-DRB1*0301 transgenic (HLA-DR3tg) mice. These mice do not express endogenous murine MHC-class II molecules and therefore serve as an ideal tool to determine peptide immunogenicity. In two experiments HLA-DR3tg mice (n=2) received a subcutaneous injection of TRP-2-derived candidate peptides (Figure 2) emulsified in CFA. Control mice were treated with CFA emulsions containing only PBS. After ten days, draining lymph nodes were harvested and ex vivo peptide reactivity of T cells was measured by an IFN-γ ELISA 48 hours after recall peptide stimulation.

Mice immunized with the TRP-2₆₀₋₇₄ peptide exhibited peptide-specific T cell responses whereas incubation of lymph node cells with an irrelevant peptide sequence did not induce any T cell stimulation. Lymph node cells from mice immunized with peptide TRP2₂₂₃₋₂₃₇ did not exhibit any peptide-reactivity. Furthermore, peptide TRP-2₃₉₈₋₄₁₃ turned out to be non-immunogenic in DR3tg mice.

### Example 4

### Protein immunisation of HLA-DRB1*0301 transgenic mice

Mice were immunized with recombinant TRP-2 protein (10-15µg/ml) emulsified in CFA. T cells from protein-vaccinated mice were screened for their peptide reactivity directly ex vivo by IFN-γ ELISA, 72 hours after recall stimulation of lymph node cells with peptide (Figure 3). Incubation of lymph node cells with peptide TRP-2₂₂₃₋₂₃₇ did not lead to any detectable cytokine release. In contrast, stimulation with peptide TRP2₆₀₋₇₄ induced IFN-γ secretion in lymph node cultures, indicating that this sequence is processed from recombinant TRP-2 protein thereby verifying TRP-2₆₀₋₇₄ as an T cell epitope.

To determine if TRP-2₆₀₋₇₄ and TRP-2₂₂₃₋₂₃₇ sequences are processed from global TRP-2 antigen, HLA-DR3tg mice were immunized with recombinant TRP-2 protein emulsified in CFA. Two different doses of recombinant protein, 10 µg (Figure 3A) and 15 µg (Figure 3B), respectively, were applied. T cells from protein vaccinated mice were screened for their peptide reactivity directly ex *vivo* by IFN-γ ELISA, 48 (Figure 3A) or 72 (Figure 3B) hours after recall stimulation of lymph node cells with peptide. In both experiments, incubation of lymph node cells with peptide TRP-2₂₂₃₋₂₃₇ did not lead to any detectable cytokine release, indicating that this sequence is probably not or not efficiently processed from recombinant TRP-2 protein. By contrast, stimulation with peptide TRP-2₆₀₋₇₄ induced IFN-γ secretion in lymph node cultures from five out of six protein immunized mice, indicating that this sequence is processed from recombinant TRP-2 protein thereby verifying TRP-2₆₀₋₇₄ as a TRP-2 derived epitope.

### Example 5

### In-vitro induction of peptide-specific T cell reactivity

The human TRP-2₆₀₋₇₄ epitope (QCTEVRADTRPWSGP) and its corresponding murine sequence (mTRP-2₆₀₋₇₄ QCAEVQTDTRPWSGP), although highly homologous, are not absolute identical in their amino acid sequences which might facilitate the generation TRP-2₆₀₋₇₄ reactive T cells in HLA-DR3tg mice. On the other hand, tolerance against the TRP-2 autoantigen might prevent the generation of TRP-2₆₀₋₇₄ reactive T cells in humans. It was determined if TRP-2₆₀₋₇₄ reactive T cells could also be activated in PBMC cultures from the peripheral blood of normal HLA-DRB1*0301 donors. Peptide-loaded mature dentritic cells were used to primarily stimulate autologous PBMC. After 3 rounds of re-stimulation T cell cultures were tested for their TRP-2₆₀₋₇₄ reactivity (Figure 5). Therefore T cells were incubated with peptide-loaded T2.DR3 target cells, derived from T2 transfected with the HLA-DRB1*0301 encoding cDNA. Bulk T cells from one donor specifically recognized TRP-2₆₀₋₇₄-loaded target cells. Since T2.DR3 cells do not express any other HLA-class II molecules, this recognition had to be HLA-DRB1*0301 restricted.

A number of HLA-class II restricted tumor epitopes have been demonstrated to exhibit binding capacitiy to more than one HLA molecule. Therefore the binding affinity of peptide TRP-2₆₀₋₇₄ to the HLA-DRB1*0401 molecule was analysed in a competitive binding assay employing the influenza virus hemagglutinin peptide (HA₃₀₇₋₃₁₉) as an already known control HLA-DRB1*0401 binder. As demonstrated in Figure 6 the TRP-2₆₀₋₇₄ peptide has the capability to bind to the HLA-DRB1*0401 molecule.

### Example 6

### Detection of memory TRP-2₆₀₋₇₄ -specific T cell responses in the peripheral blood of HLA-DRB1*0301⁺ melanoma patients

In vivo sensitized CTL have been isolated repeatedly from the peripheral blood of a variety of melanoma patients, leading to the assumption that also spontaneous antigen-specific CD4⁺ T cells might be detectable. Therefore PBMC from two HLA-DRB1*0301⁺ melanoma patients and four healthy HLA-DRB1*0301 blood donors were analysed for the presence of TRP-2₆₀₋₇₄-reactive T cells. PBMC were incubated with TRP-2₆₀₋₇₄ and TRP-2₂₂₃₋₂₃₇ peptides, respectively, over a period of ten days. This single round of *in vitro* stimulation recalls only memory responses. Cells were then analyzed for peptide-specific cytokine release in an ELISPOT assay. TRP-2₆₀₋₇₄ specific T cell stimulation could clearly be detected in PBMC cultures from patient HB (Figure 4) whereas peptide TRP-2₂₂₃₋₂₃₇ did not induce any cytokine release. Memory T cell responses could not be detected in cultures from the second melanoma patient and from any of the healthy donors.

### Example 7

### Detection of memory TRP-2₆₀₋₇₄-specific T cell responses in the peripheral blood of HLA-DRB1*0401⁺ melanoma patients

To determine, if TRP-2₆₀₋₇₄-specific T cell reactivity can be detected in the peripheral blood of HLA-DRB1*0401 melanoma patients, PBMC from four patients were incubated over ten days with the natural TRP-2₆₀₋₇₄ epitope or with an epitope variant TRP-2₆₀₋₇₄YT, postulated to exhibit stronger binding affinity to the HLA-DRB1*0401 molecules. Peptide-reactive T cells could be detected in patient KM (Figure 7). T cell reactivity against the variant TRP-2₆₀₋₇₄YT epitope was stronger then T cell stimulation obtained by the natural ligand. No T cell reactivity was detectable against the TRP-2₂₂₃₋₂₃₇ sequence which also exhibits HLA-DRB1*0401 binding capacity.

### Example 8

### In vitro induction of peptide-specific T cell reactivity

It was determined if TRP-2₆₀₋₇₄YT reactive T cells could also be activated in PBMC cultures from the peripheral blood of normal HLA-DRB1*0401 donors. Peptide-loaded mature dendritic cells from donor BF 302239320 were used to primarily stimulate autologous PBMC. After 3 rounds of restimulation T cell reactivity was tested against T2.DR4 transfectants loaded either with the TRP-2₆₀₋₇₄YT peptide or the natural TRP-2₆₀₋₇₄ epitope sequence (Figure 8). Interestingly, TRP-2₆₀₋₇₄ YT primed T cells recognized TRP-2₆₀₋₇₄YT-loaded target cells and cross-reacted with TRP-2₆₀₋₇₄-loaded T2.DR4 cells, indicating that the variant peptide can be employed to efficiently stimulate T cells recognizing the natural sequence.

### Example 9

### Recognition of tumor cells

TRP-2₆₀₋₇₄YT specific T cells from donor BF 302239320 were analysed for their capability to recognize HLA-DRB1*0401⁺, TRP-2 expressing tumor cells. Coincubation with UKRV-Mel-17 cells (DR4+, TRP-2+) induced TNF secretion by TRP-2₆₀₋₇₄YT specific T cells, whereas coincubation of T cells with SK-Mel-37 tumor cells (DR4⁻, TRP-2⁺) did not lead to any T cell stimulation (Figure 9).

### Example 10

### Analysis of the HLA-DRB1*0301 binding capacity of TRP-1 derived sequences

The screening of the human TRP-1 sequence for potential HLA-DRB1*0301 restricted epitopes by computer algorithms led to the selection of three peptide sequences as candidate epitopes: TRP-1₁₃₂₋₁₄₆, TRP-1₁₆₆₋₁₈₀ and TRP-1₂₂₇₋₂₄₁. The capacity of the sequences to bind to the HLA-DRB1*0301 molecule was analysed in a competitive binding assay employing a Tetanus Toxin peptide (TT₈₂₉₋₈₄₃) as an already known control HLA-DRB1*0301 binder. As demonstrated in Figure 10 the peptides TRP-1₁₆₆₋₁₈₀ and TRP-1₂₂₇₋₂₄₁ have the capability to bind to the HLA-DRB1*0301 molecule. In contrast, the sequence TRP-1₁₃₂₋₁₄₆ did not exhibit any affinity for the HLA-DRB1*0301 molecule.

### Example 11

### Analysis of peptide immunogenicity in HLA-DRB1*0301 transgenic mice

Based on their HLA-DR binding capacity, the peptides TRP-1₁₆₆₋₁₈₀ and TRP-1₂₂₇₋₂₄₁ were selected for peptide immunization of HLA-DR3tg mice. Mice received subcutaneous injections of 100 µg peptide emulsified in CFA. Control mice were treated with CFA mixed with PBS. After ten days draining lymph node cells were harvested and analysed for peptide-specific T cell reactivity by ex vivo IFN-γ ELISA, 48 hours after recall peptide stimulation (Figure 11). Mice immunized with the TRP-1₁₆₆₋₁₈₀ and TRP-1₂₂₇₋₂₄₁ peptides, respectively, exhibited peptide-specific T cell responses whereas incubation of lymph node cells with an irrelevant peptide sequence did not induce any T cell stimulation. Therefore both peptides are clearly immunogenic in HLA-DR3tg mice.

### Example 12

### Memory TRP-1₁₆₆₋₁₈₀-specific T cell responses can be detected in the peripheral blood of HLA-DRB1*0301⁺ melanoma patients

To determine, if TRP-1₁₆₆₋₁₈₀ or TRP-1₂₂₇₋₂₄₁ specific T cells can be detected in the peripheral blood of HLA-DRB1*0301 melanoma patients, PBMC from two patients were incubated over ten days with the peptides. In addition, PBMC from four normal HLA-DRB1*0301 blood donors were included in the assay. TRP-1₁₆₆₋₁₈₀ peptide-reactive T cells could be detected in PBMC cultures from patient BH (Figure 12), verifying this sequence as a target of spontaneous T cell responses. In contrast, no specific T cell reactivity could be detected against the TRP-1₂₂₇₋₂₄₁ sequence.

### Example 13

### Analysis of HLA-DRB1*0401 binding capacity of TRP-1 and TRP-2 derived sequences

The screening of the human TRP-1 and TRP-2 sequences for potential HLA-DRB1*0401 restricted epitopes led to the selection of two peptide sequences as candidate epitopes: TRP-1₄₁₅₋₄₂₇ and TRP-2₂₈₈₋₃₀₂. The capacity of the sequences to bind to the HLA-DRB1*0401 molecule was analysed in a competitive binding assay employing the influenza virus hemagglutinin peptide (HA₃₀₇₋₃₁₉) as an already known control HLA-DRB1*0401 binder. As demonstrated in Figure 13 both peptides have the capability to bind to the HLA-DRB1*0401 molecule, although binding affinity of the TRP-1₄₁₅₋₄₂₇ sequence is much stronger.

### Example 14

### Memory TRP-1₄₁₅₋₄₂₇-specific T cell responses can be detected in the peripheral blood of HLA-DRB1*0401⁺ melanoma patients

To determine, if TRP-1₄₁₅₋₄₂₇ or TRP-2₂₈₈₋₃₀₂ specific T cell reactivity can be detected in the peripheral blood of HLA-DRB1*0401 melanoma patients, PBMC from four patients were incubated over ten days with the peptides. TRP-1₄₁₅₋₄₂₇ peptide-reactive T cells could be detected in PBMC cultures from patient GM (Figure 14), verifying this sequence as a target of spontaneous T cell responses. In contrast, no T cell reactivity could be detected against the TRP-2₂₈₈₋₃₀₂ sequence.

### Example 15

### Analysis of peptide immunogenicity in HLA-DQ8 transgenic mice

The screening of the human TRP-1 and TRP-2 sequences for potential HLA-DQ8 restricted epitopes led to the selection of three peptide sequences as candidate epitopes: TRP-1₁₇₋₂₉, TRP-1₂₄₆₋₂₆₀ and TRP-2₂₄₂₋₂₅₆. The capacity of the sequences to bind to the HLA-DQ8 molecules could not be analysed in a competitive binding assay due to the lack of a well defined control HLA-DQ8 binder. Therefore peptide immunogenicity was directly analysed by peptide immunization of HLA-DQ8tg mice. Mice received subcutaneous injections of 100 µg peptide emulsified in CFA. Control mice were treated with CFA mixed with PBS. After ten days draining lymph node cells were harvested and analysed for peptide-specific T cell reactivity by ex vivo IFN-γ ELISA, 48 hours after recall peptide stimulation (Figure 15). Only mice immunized with the TRP-2₂₄₂₋₂₅₆ peptide exhibited peptide-specific T cell responses indicating that this peptide is clearly immunogenic in HLA-DQ8tg mice.

### Example 16

### Memory TRP-2₂₄₂₋₂₅₆-specific T cell responses can be detected in the peripheral blood of HLA-DQ2⁺ melanoma patients

Interestingly, HLA-DQ8 and HLA-DQ2 molecules have recently been described to present a largely overlapping peptide repertoire. To determine, if TRP-2₂₄₂₋₂₅₆ specific T cell reactivity can be detected in the peripheral blood from melanoma patients, PBMC from the HLA-DQ2 melanoma patient BH were incubated over ten days with the peptide. TRP-2₂₄₂₋₂₅₆ peptide-reactive T cells could be detected in PBMC cultures from this patient (Figure 16), verifying this sequence as a target of spontaneous T cell responses.

## Claims

1. A TRP-2 peptide capable of activating CD4⁺ T helper cells (Th) against a TRP-2⁺ and/or TRP-1⁺ tumor, wherein said peptide comprises
(a) the amino acid sequences QCTEVRADTRPWSGP (human TRP-2₆₀₋₇₄), QCTEYRADTTPWSGP (TRP-2₆₀₋₇₄YT, epitope variant of human TRP-2₆₀₋₇₄), LPYWNFATGRNECDV (human TRP-2₂₄₂₋₂₅₆), SEEILGPDGNTPQFE (human TRP-1₁₆₆₋₁₈₀), LRRYNADISTFPL (human TRP-1₄₁₅₋₄₂₇)
(b) an amino acid sequence which differs from an amino acid sequence from (a) by one or more amino acid substitutions
(c) a peptide having an amino acid sequence which shows at least 50% identity to the amino acid sequence of the peptide of (a) or (b);
(d) a peptide having an amino acid sequence which shows at least 70% identity to the amino acid sequence of the peptide of (a) or (b);
(e) a peptide having an amino acid sequence which shows at least 90% identity to the amino acid sequence of the peptide of (a) or (b); or
(f) a peptide which is a fragment of the peptide of (a), (b), (c) , (d) or (e);
wherein the peptides of (b), (c), (d), (e) or (f) are still capable of activating CD4⁺ T helper cells (Th) against a TRP-2⁺ and/or TRP-1⁺ tumor.

2. The peptide of claim 1, wherein said tumor is a malignant melanoma or glioblastoma.

3. A fusion protein comprising a peptide of claim 1 or 2.

4. A polynucleotide encoding the peptide of claim 1 or 2, or the fusion protein of claim 3.

5. An expression vector containing the polynucleotide of claim 4.

6. A host cell transformed or transfected with an expression vector of claim 5.

7. An antibody that specifically binds to a peptide of claim 1 or 2.

8. A pharmaceutical composition containing a peptide of claim 1 or 2, a polynucleotide of claim 4, an expression vector of claim 5 or an antibody of claim 7.

9. Use of a peptide according to claim 1 or 2, a polynucleotide of claim 4 or an expression vector of claim 5 for the preparation of a medicament for the treatment of a TRP-2⁺ and/or TRP-1⁺tumor.

10. Use according to claim 9, wherein said treatment is stimulation of an immune response.

11. Use of a peptide according to claim 1 or 2, a polynucleotide of claim 4 or an expression vector of claim 5 for the preparation of a medicament for the vaccination of tumor patients with DC.

12. Use of a peptide according to claim 1 or 2, a polynucleotide of claim 4 or an expression vector of claim 5 for the in vitro generation of autologeous antigen-specific Th.

13. Use of a polynucleotide of claim 4 or an expression vector of claim 5 for the preparation of a polypeptide-DNA-vaccine for treatment of tumors.

14. A Th cell population comprising Th cells that had been activated with a peptide of claim 1 or 2, or a fusion protein of claim 3.

15. A diagnostic composition containing a peptide of claim 1 or 2, a fusion protein of claim 3 or an antibody of claim 7.

16. Use of a peptide of claim 1 or 2, or a fusion protein of claim 3 for the preparation of a diagnostic composition for monitoring an Th immune response against a TRP-2⁺ and/or TRP-1⁺ tumor.

17. Use of an antibody of claim 7 for the preparation of a diagnostic composition for detecting the presence of a TRP-2⁺ and/or TRP-1⁺ tumor in a patient.

18. A diagnostic kit comprising a peptide of claim 1 or 2, a fusion protein of claim 3 or an antibody according to claim 7 and a detection reagent.

19. The diagnostic kit of claim 18, wherein said detection reagent comprises a reporter group.
